# EUROPÄISCHE PATENTANMELDUNG

(11) **EP 0 567 724 A1**
(43) Veröffentlichungstag der Anmeldung: **03.11.1993**
(21) Anmeldenummer: 93101004.5
(22) Anmeldetag: 22.01.1993
(51) Int. Cl.: A61F 7/10, A61F 7/02

(54) **Kompresse**

(30) Priorität: 02.04.1992 DE 9204540 U
(71) Anmelder: QUINTA GESELLSCHAFT FÜR PRODUKT-MARKETING mbH, 79104 Freiburg (DE)
(72) Erfinder: Behre, Hans Jürgen, W-7801 Sölden (DE)
(74) Vertreter: Urner, Peter, Dipl.-Phys.

(57) **Zusammenfassung**

Die Erfindung betrifft eine Kompresse aus flexiblem Material, die um einen Gegenstand herumwickelbar ist, um diesen zu temperieren, insbesondere zu kühlen. Dabei ist die Kompresse hüllenartig ausgebildet und in eine Mehrzahl von Kammern (3a - 3f; 4a - 4e) unterteilt, die jeweils zur Aufnahme eines Temperiermediums dienen, welches z. B. in einer Folie eingeschweißt ist. Insbesondere eignet sich die Kompresse zur Behandlung von venösen Beinleiden und zur Thromboseprophylaxe und kann auf einem Stützstrumpf zu liegen kommen, um gleichzeitig den zu behandelnden Körperteil mechanisch belasten und kühlen zu können.

## Beschreibung

Die Erfindung betrifft eine Kompresse gemäß dem Oberbegriff des Schutzanspruchs 1.

Eine Kompresse dieser Art kann auf den verschiedensten Gebieten zum Einsatz kommen, beispielsweise im Bereich der Therapie, der Chirurgie, und dergleichen. Sie besteht üblicherweise aus flexiblem Material, das um einen Gegenstand herumwickelbar ist, um diesen zu temperieren. Dieser Gegenstand kann z. B. ein Arm oder Bein eines Patienten sein.

So hat sich etwa bei der Thromboseprophylaxe gezeigt, daß neben dem Einsatz von Stützstrümpfen zur Gefäßverengung auch eine Kälteapplikation von Vorteil ist, um zu besseren Therapieergebnissen zu gelangen. Eine geeignete Applikationshilfe war allerdings bisher nicht vorhanden.

Der Erfindung liegt die Aufgabe zugrunde, eine Kompresse der eingangs genannten Art zu schaffen, die sich insbesondere fuhr den Einsatz bei venösen Beinleiden und zur Thromboseprophylaxe eignet sowie leicht handhabbar ist.

Die Lösung dergestellten Aufgabe ist im kennzeichnenden Teil des Schutzanspruchs 1 angegeben. Vorteilhafte Ausgestaltungen der Erfindung sind den Unteransprüchen zu entnehmen.

Eine Kompresse nach der Erfindung zeichnet sich dadurch aus, daß sie hüllenartig ausgebildet und mit einem Temperiermedium gefüllt ist.

Wird die Kompresse zur Behandlung von Armen oder Beinen verwendet, so enthält sie als Temperiermedium ein Kühlmedium. Dabei wird die Kompresse um Arm oder Bein eines zu behandelnden Patienten herumgewickelt, und zwar direkt auf den Arm oder das Bein, oder auf einen diesen Körperteil umgebenden Stützstrumpf, so daß nunmehr eine kombinierte Stütz-Kühlwirkung erzielt wird, die zu ausgezeichneten Therapieergebnissen führt.

Das Kühlmedium weist eine pastenartige Konsistenz auf, so daß es sich an Arm oder Bein anschmiegen kann, wenn die Kompresse diesen Körperteil manschettenartig umgibt.

Das Kühlmedium ist vorzugsweise eine wachsartige Masse bzw. eine Paraffinmasse, in welcher Wasserperlchen eingebettet sind. Nach Herunterkühlen dieser Masse sind etwa 50 Prozent mikrokristallines Eis vorhanden, wodurch eine lang andauernde und gleichmäßige Kühlung erzielt wird. Eine derartige Masse ist bereits Gegenstand des Deutschen Patents P 31 41 191.

Nach einer vorteilhaften Ausgestaltung der Erfindung weist die Kompresse eine in Kompressen-Wickelrichtung uneinheitliche Breite auf. Sie kann daher speziell so zugeschnitten sein, daß sie sowohl den oberen als auch den unteren Arm- bzw. Beinbereich vollständig umgreift, um zu verhindern, daß es zu freibleibenden oder zu sich überlappenden Bereichen kommt.

Nach einer anderen vorteilhaften Ausgestaltung der Erfindung ist die Kompresse in einzelne Kammern unterteilt, in denen sichjeweils das Temperier- bzw. Kühlmedium befindet. Hierdurch wird für eine gleichmäßigere Verteilung des Temperiermediums im Innern der Kompresse gesorgt, was zu einer noch gleichmäßigeren Temperierung des umwickelten Gegenstands führt.

Beispielsweise können sich die Kammern senkrecht zur Kompressen-Wickelrichtung erstrecken, also im Falle eines zu kühlenden Beins in Beinlängsrichtung, wobei die Kammern dann in Kompressen-Wickelrichtung parallel nebeneinanderliegend angeordnet sind. Dabei können auch Gruppen von Kammern in Kompressen-Wickelrichtung gegeneinander versetzt sein, um die Temperier- bzw. Kühlwirkung der Kompresse noch weiter zu vergleichmäßigen.

Nach einer sehr vorteilhaften Weiterbildung der Erfindung befindet sich das Temperiermedium in Taschen bzw. Beuteln, deren Größe an die Kammergröße angepaßt ist. Die Taschen können beispielsweise aus verschweißter Folie hergestellt sein. Sie lassen sich dann in einfacher Weise in die Kammern einsetzen und nach Gebrauch der Kompresse wieder aus dieser herausnehmen, um in einem Kühlaggregat erneut heruntergekühlt werden zu können.

Dabei können die Kammern vorzugsweise mit Verschlüssen versehen sein, um ein Herausfallen der Taschen aus den Kammern und damit verbundene Druckveränderungen während des Gebrauchs der Kompresse zu vermeiden. Vorzugsweise sind die Verschlüsse Reißverschlüsse, wobei sich auch mehrere nebeneinanderliegende Kammern mit ein und demselben Reißverschluß verschließen lassen.

Die Kompresse selbst ist mit einem Kompressenverschluß versehen, mit dessen Hilfe sie fest am umwickelten Gegenstand gehalten wird. Der Kompressenverschluß kann beispielsweise ein Klettverschluß sein. Dieser läßt sich vorteilhaft durch Verschlußstreifen bilden, die parallel zur Kompressen-Wickelrichtung verlaufen.

Das flexible Material der Kompresse besteht vorzugsweise aus textilem Material, das atmungsaktiv und damit hautfreundlich ist. Insbesondere kann es sich hier um Baumwolle oder Leinen handeln.

Die einandergegenüberliegenden Bahnen des flexiblen bzw. textilen Materials lassen sich dabei zur Bildung der Kammern zusammennähen, so daß im Bereich der Kammertrennwände keine Flexibilitätseinbußen befürchtet zu werden brauchen. In diesen Bereichen kann die Haut darüber hinaus atmen, da sie hier nicht durch Kühlmedium abgedeckt wird.

Die Erfindung wird nachfolgend unter Bezugnahme auf die Zeichnung näher beschrieben. Es zeigen:
**Fig. 1** eine Kompresse mit zwei Gruppen von gegeneinander versetzt angeordneten Kammern und
**Fig. 2** die Kompresse nach Fig. 1 mit herausgenommenen und seitlich positionierten Folientaschen für das Temperiermedium.

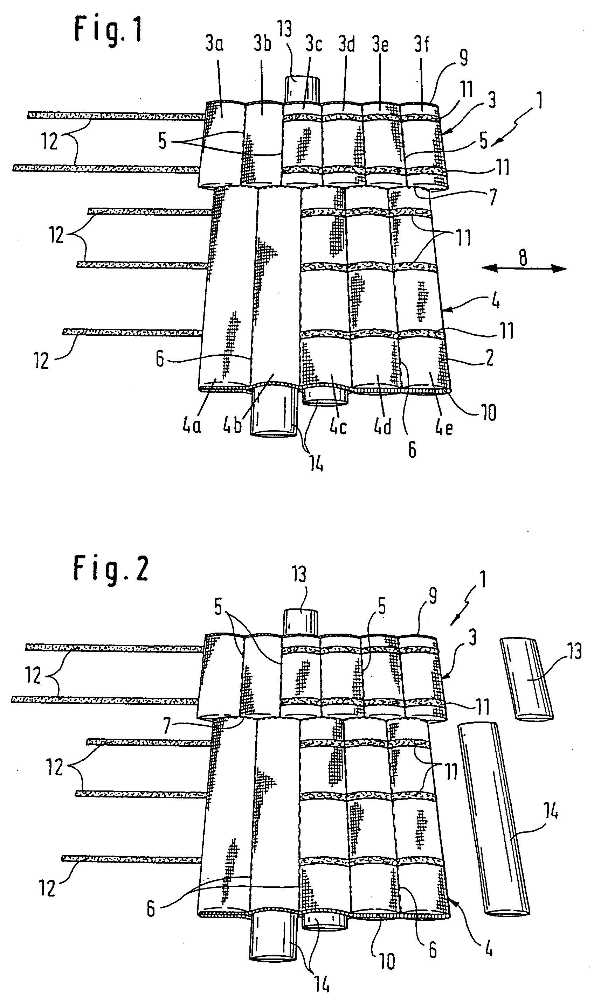

Die Kompresse nach Fig. 1 ist mit dem Bezugszeichen 1 versehen und besteht aus einer Stoffhülle 2, beispielsweise aus einer Baumwollhülle. Die einander gegenüberliegenden Wände der Stoffhülle 2 sind so miteinander vernäht, daß eine erste Gruppe 3 und eine zweite Gruppe 4 von parallel zueinander verlaufenden Kammern 3a bis 3f und 4a bis 4e entstehen. Die zwischen den Kammern verlaufenden und als Trennwände dienenden Nähte tragen jeweils die Bezugszeichen 5 und 6. Darüber hinaus sind die erste Gruppe 3 und die zweite Gruppe 4 durch eine in Fig. 1 horizontal verlaufende Naht 7 voneinander getrennt.

Wie zu erkennen ist, sind die Kammern 3a bis 3f der ersten Gruppe 3 gegenüber den Kammern 4a bis 4e der zweiten Gruppe 4 in Längsrichtung der Horizontalnaht 7 gegeneinander versetzt, wobei zur ersten Gruppe 3 sechs Kammern gehören, während zur zweiten Gruppe 4 fünf Kammern gehören. Sämtliche Kammern sind in Längsrichtung der Horizontalnaht 7 gleich breit, während senkrecht dazu die Kammern der ersten Gruppe 3 kürzer sind als die Kammern der zweiten Gruppe 4. Alle Kammern sind mit anderen Worten schlauchartig ausgebildet. Die Schlauchlängsrichtung verläuft senkrecht zur Horizontalnaht 7, die ihrerseits in Kompressen-Wickelrichtung 8 verläuft.

Die Kammern 3a bis 3f der ersten Gruppe 3 und die Kammern 4a bis 4e der zweiten Gruppe 4 sind jeweils an ihrer freien Stirnseite mit einem Reißverschluß 9, 10 verschließbar, der sich über die gesamte in Kompressen-Wickelrichtung 8 liegende Breite der Kompresse 1 erstreckt. Die Kompresse 1 selbst trägt an ihrer außenliegenden Oberfläche einen Klettverschluß, der aus mehreren in Kompressen-Wickelrichtung 8 und im Abstand voneinander liegenden Haltestreifen 11 besteht, die über ihre gesamte Länge mit der Stoffhülle 2 vernäht sind. Auf gleicher Höhe liegende Verschlußfahnen 12 lassen sich mit den Haltestreifen 11 in Kontakt bringen, um den Klettverschluß zu schließen.

Die Fig. 1 und 2 lassen deutlich erkennen, daß in die Kammern 3a bis 3f der ersten Gruppe 3 und in die Kammern 4a bis 4e der zweiten Gruppe 4 Taschen 13, 14 stirnseitig hineingeschoben werden können, und zwar nach Öffnen der Reißverschlüsse 9 und 10. Diese Taschen 13, 14 sind in Fig. 2 nochmals separat dargestellt und weisen eine Größe auf, die der Größe der jeweiligen Kammern entspricht. Sie füllen mit anderen Worten die Kammern vollständig aus, wenn sie in diese hineingeschoben worden sind.

Die genannten Taschen 13, 14 bestehen aus einer Folie, die an ihren Rändern verschweißt ist. Innerhalb der Taschen 13, 14 befindet sich das Temperiermedium, beispielsweise eine pastenförmige Kühlmasse. Bei Anwendung der Kompresse zu Therapiezwecken wird diese Masse auf Minustemperaturen bis zu -20 Grad Celsius heruntergekühlt und zeigt dann eine über längere Zeit konstant bleibende Kühlwirkung.

Wird die Kompresse nach den Fig. 1 und 2 in Benutzung genommen, so werden zunächst die Taschen 13, 14 heruntergekühlt und in die jeweiligen Kammern 3a bis 3f der ersten Kammergruppe 3 und die Kammern 4a bis 4e der zweiten Kammergruppe 4 stirnseitig hineingeschoben. Bei der therapeutischen Behandlung wird die Kompresse 1 anschließend um den zu behandelnden Arm bzw. das zu behandelnde Bein herumgelegt, wobei die Längsrichtung der Kammern in Arm- bzw. Beinlängsrichtung weist. Die Kompresse 1 kann dabei auf einem Stützstrumpf zu liegen kommen, den der Arm bzw. das Bein bereits trägt. Sie wird nach Herumlegen um diesen Körperteil mit Hilfe des Klettverschlusses 11, 12 verschlossen, wobei sich das Kühlmedium mit konstantem Druck an den Körperteil anschmiegt.

Die erste Kammergruppe 3, die in Kompressen-Wickelrichtung 8 länger ist als die zweite Kammergruppe 4, kann dabei im Bereich des Oberarms oder des Oberschenkels zu liegen kommen.

## Patentansprüche

1. Kompresse aus flexiblem Material, die um einen Gegenstand herumwickelbar ist, um diesen zu temperieren, **dadurch gekennzeichnet**, daß sie hüllenartig ausgebildet und mit einem Temperiermedium gefüllt ist.

2. Kompresse nach Anspruch 1, **dadurch gekennzeichnet**, daß sie eine in Kompressen-Wickelrichtung (8) uneinheitliche Breite aufweist.

3. Kompresse nach Anspruch 1 oder 2, **dadurch gekennzeichnet**, daß sie in einzelne Kammern (3a - 3f; 4a -4e) unterteilt ist, in denen sich jeweils das Temperiermedium befindet.

4. Kompresse nach Anspruch 3, **dadurch gekennzeichnet**, daß sich die Kammern (3a - 3f; 4a - 4e) senkrecht zur Kompressen-Wickelrichtung (8) erstrecken und in Kompressen-Wickelrichtung (8) parallel nebeneinanderliegend angeordnet sind.

5. Kompresse nach Anspruch 3 oder 4, **dadurch gekennzeichnet**, daß Gruppen (3; 4) von Kammern (3a - 3f; 4a -4e) in Kompressen-Wickelrichtung (8) gegeneinander versetzt sind.

6. Kompresse nach einem der Ansprüche 3 bis 5, **dadurch gekennzeichnet**, daß sich das Temperiermedium in Taschen (13; 14) befindet, deren Größe an die Kammergröße angepaßt ist.

7. Kompresse nach Anspruch 6, **dadurch gekennzeichnet**, daß die Taschen (13; 14) aus verschweißter Folie gebildet sind.

8. Kompresse nach einem der Ansprüche 3 bis 7, **dadurch gekennzeichnet**, daß die Kammern (3a - 3f; 4a - 4e) mit Verschlüssen (9; 10) versehen sind.

9. Kompresse nach Anspruch 8, **dadurch gekennzeichnet**, daß mehrere Kammern mit einem Verschluß (9; 10) verschließbar sind.

10. Kompresse nach Anspruch 8 oder 9, **dadurch gekennzeichnet**, daß der Verschluß ein Reißverschluß (9; 10) ist.

11. Kompresse nach einem der Ansprüche 1 bis 10, **dadurch gekennzeichnet**, daß sie mit einem Kompressenverschluß (11, 12) versehen ist.

12. Kompresse nach Anspruch 11, **dadurch gekennzeichnet**, daß der Kompressenverschluß ein Klettverschluß ist.

13. Kompresse nach einem der Ansprüche 1 bis 12, **dadurch gekennzeichnet**, daß das flexible Material textiles Material ist.

14. Kompresse nach einem der Ansprüche 3 bis 13, **dadurch gekennzeichnet**, daß einander gegenüberliegende Bahnen des flexiblen Materials zur Bildung der Kammern (3a - 3f; 4a - 4e) zusammengenäht sind.

15. Kompresse nach einem der Ansprüche 1 bis 14, **dadurch gekennzeichnet**, daß das Temperiermedium ein Kühlmedium ist.

16. Kompresse nach Anspruch 15, **dadurch gekennzeichnet**, daß das Kühlmedium eine pastenartige Konsistenz aufweist.

17. Kompresse nach Anspruch 16, **dadurch gekennzeichnet**, daß das Kühlmedium in Wachs eingebettete Wasserperlchen enthält.
